(19) Europäisches Patentamt
European Patent Office
Office européen des brevets

(11) EP 2 804 880 B1

(12) **FASCICULE DE BREVET EUROPEEN**

(45) Date de publication et mention de la délivrance du brevet:
**06.07.2016 Bulletin 2016/27**

(21) Numéro de dépôt: **13705213.0**

(22) Date de dépôt: **14.01.2013**

(51) Int Cl.:
***C08F 220/28*** (2006.01) ***C08F 226/04*** (2006.01)
***A61K 8/81*** (2006.01) ***A61Q 5/00*** (2006.01)
***C08F 283/06*** (2006.01) ***C11D 3/37*** (2006.01)

(86) Numéro de dépôt international:
**PCT/FR2013/050087**

(87) Numéro de publication internationale:
**WO 2013/107976 (25.07.2013 Gazette 2013/30)**

(54) **NOUVEAUX POLYMERES PEIGNES UTILISABLES EN COSMETIQUE ET DETERGENCE**

NEUARTIGE KAMMPOLYMERE ZUR VERWENDUNG IN KOSMETIKARTIKELN UND REINIGUNGSMITTELN

NOVEL COMB POLYMERS WHICH CAN BE USED IN COSMETICS AND DETERGENCY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.01.2012 FR 1250380**

(43) Date de publication de la demande:
**26.11.2014 Bulletin 2014/48**

(73) Titulaire: **S.P.C.M. SA**
**42160 Andrézieux Bouthéon (FR)**

(72) Inventeurs:
- **BLONDEL, Frédéric**
**F-42600 Lezigneux (FR)**
- **SANNA, Antonin**
**F-42000 Saint Etienne (FR)**

(74) Mandataire: **Cabinet Laurent & Charras**
**Le Contemporain**
**50 Chemin de la Bruyère**
**69574 Dardilly Cedex (FR)**

(56) Documents cités:
**AU-B2- 2004 200 189 FR-A1- 2 962 034**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 804 880 B1

Printed by Jouve, 75001 PARIS (FR)

## Description

La présente invention concerne le secteur technique des polymères de structure "peigne". Plus précisément, l'invention concerne le secteur technique des polymères peignes cationiques ayant des chaines pendantes hydrophiles. Ces polymères peignes trouvent des applications, notamment dans les domaines de la cosmétique et de la détergence.

Un polymère "peigne" présente une structure similaire à celle d'un peigne. En d'autres termes, il comporte une chaîne principale sur laquelle sont fixées des chaînes latérales qui peuvent être de nature différente et de longueur variable. Par exemple, ces chaînes latérales peuvent présenter des propriétés hydrophiles et/ou hydrophobes. Elles peuvent notamment être de type éthylène oxyde, propylène oxyde, alkyle, etc..., avec des longueurs de 2 à 500 motifs et de préférence de 5 à 200 motifs dans le cas d'une chaîne pendante de type polyéthylèneoxyde.

Les polymères peignes de l'art antérieur comprennent notamment les polymères à base de motifs (méth)acrylate de polyéthylène glycol (PEGMA), et de motifs cationiques.

Le document EP372546 décrit des copolymères à base de PEGMA, de monomères de type (méth)acrylamides d'alkyles en $C_1$-$C_8$, et éventuellement des monomères cationiques.

Le document JP2002-322219 décrit des polymères contenant des motifs PEGMA en association avec des monomères hydrophobes à base de polypropylène glycol (PPO) ou de polyoxyde de tétraméthylène, et des monomères cationiques.

Le document JP2003-055164 décrit des polymères réticulés contenant des motifs du type PEGMA; toutefois, ces polymères sont réticulés rendant ainsi le contrôle de leur synthèse plus délicat.

Le document JP2000-302649 décrit aussi une composition capillaire comprenant un polymère à base de monomères cationiques présentant des groupements amines quaternaires, de monomères à groupement polyéther, notamment de type PEG (polyéthylène glycol) ou PPO, et optionnellement de monomères hydrophobes (par exemple méthacrylate de stéaryle).

Le document JP07-285831 décrit des compositions capillaires contenant un polymère à base de monomères de type PEGMA associés à des monomères ioniques, cationiques ou amphotères, et des monomères additionnels de type (méth)acrylates d'alkyle en $C_1$-$C_{24}$, principalement hydrophobes.

Les documents EP1769011 et EP1765893 décrivent des polymères constitués majoritairement de motifs cationiques et de motifs PEGMA.

Le document WO2006/013268 décrit des polymères comprenant au moins un monomère du type (méth)acrylate de PEG associé à un monomère à caractère cationique (cationique, amphotère ou cationique et anionique).

Le document AU 2004 200 189 décrit un polymère pouvant comprendre un monomère du type acrylate de PEG associé à un monomère pouvant être cationique mais ne comprenant pas de fonction ammonium quaternaire.

Le document FR 2 962 034 décrit un polymère contenant un monomère du type méthacrylate de PEG associé à un monomère ne comprenant pas de fonction ammonium quaternaire.

Comme déjà indiqué, les domaines d'application des polymères peigne concernent notamment la cosmétique et la détergence. Ils peuvent donc être présents en tant qu'agent conditionneur dans des compositions de produits corporels et capillaires, ou d'agent d'aide à la déposition dans les lessives.

Toutefois la présence d'espèces anioniques dans les formulations finales apporte des incompatibilités avec les polymères cationiques.

L'incompatibilité entre un polymère cationique et une espèce anionique, du fait de leurs charges opposées, peut se traduire par l'apparition de précipité c'est-à-dire un agrégat insoluble résultant de la coalescence de particules colloïdales en suspension. De manière générale, la formation de précipité est bien entendu contre-indiquée dans les formulations du type shampooing, lessive, ou adoucissant.

La Demanderesse a mis au point des polymères permettant notamment de pallier les inconvénients liés aux incompatibilités entre les espèces anioniques et les polymères cationiques.

La présente invention concerne ainsi des polymères qui, une fois incorporés dans une composition cosmétique ou détergente, permettent d'éviter la formation de précipités résultant de l'attraction ionique entre deux composés de charges opposées.

Le Demandeur a mis en évidence que ce problème technique peut être résolu non seulement en limitant la proportion de monomère(s) cationique(s) dans le polymère à moins de 50% en masse, mais aussi en combinant le(s) monomère(s) cationique(s) avec au moins un monomère présentant une chaîne pendante, permettant ainsi d'ajuster la densité de charge cationique du polymère.

La présente invention a donc pour objet un copolymère hydrosoluble à motifs éthyléniques, comprenant, en pourcentage en masse par rapport à la masse totale du copolymère :

- 5 à 45% d'au moins un monomère cationique dont la cationicité provient exclusivement de fonction(s) ammonium quaternaire ;
- 50 à 95% d'au moins un monomère de formule (I) ; avantageusement de 55 à 95% ;

(I) 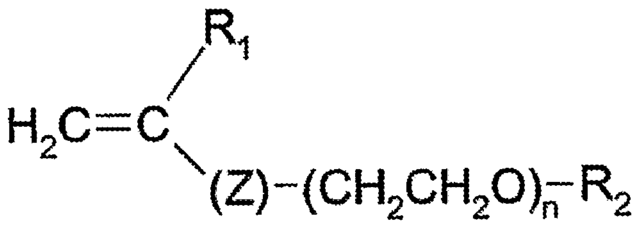

dans laquelle :

- $R_1$ est un atome d'hydrogène ou un radical méthyle,
- Z est le groupement divalent -C(=O)-O-, ou -C(=O)-NH-,
- n est un nombre entier compris entre 2 et 200;
- $R_2$ est un atome d'hydrogène ou un radical carboné, saturé ou insaturé, éventuellement aromatique, linéaire, ramifié ou cyclique, comprenant 1 à 30 atomes de carbone, comprenant de 0 à 4 hétéroatomes choisis dans le groupe comprenant O, N, et S.

De manière avantageuse, n est compris entre 7 et 45.
En outre, selon une caractéristique essentielle de l'invention, la cationicité du copolymère est avantageusement comprise entre 0.3 et 2.6 meq/g, préférentiellement entre 0.5 et 1.5 meq/g.
Par le terme « hydrosoluble », on désigne un copolymère pouvant être mis en solution aqueuse, à hauteur d'au moins 50 g/L à 25°C, sans laisser subsister de particules insolubles.
La cationicité ou densité de charge cationique correspond au nombre d'équivalent cationique par unité de masse.
En d'autres termes, dans le cas où le copolymère comprend un monomère cationique A, et un monomère non cationique B, elle est déterminée selon la formule suivante :

$$\text{Cationicité (meq/g)} = (1000 \times \%A) / (\%A \times Mw_A + \%B \times Mw_B)$$

dans laquelle :

- %A représente le pourcentage molaire du monomère cationique A ;
- %B représente le pourcentage molaire du monomère non cationique B ;
- $Mw_A$ représente la masse molaire du monomère cationique A ;
- $Mw_B$ représente la masse molaire du monomère non cationique B.

La densité de charge cationique dépend donc des proportions de monomères ainsi que de leurs masses molaires respectives. Par conséquent, à ratio de monomères équivalents, deux polymères ne présenteront pas nécessairement la même densité de charge cationique eu égard à la masse molaire de chacun des monomères.
Selon un mode de réalisation particulier, le copolymère est constitué de, en pourcentage en masse par rapport à la masse totale du copolymère :

- 5 à 45% d'au moins un monomère cationique dont la cationicité provient exclusivement de fonction(s) ammonium quaternaire ;
- 55 à 95% d'au moins un monomère de formule (I).

Selon un mode de réalisation particulier, le copolymère présente des charges cationiques provenant uniquement de fonctions ammonium quaternaire. En d'autres termes, selon ce mode de réalisation particulier, la cationicité du ou des monomère(s) cationiques contenus dans le copolymère est exclusivement due à la présence de fonctions ammonium quaternaire. Selon ce mode de réalisation, la totalité des charges cationiques du copolymère provient de fonctions ammonium quaternaire.
Selon un mode de réalisation préféré, dans le monomère de formule (I), $R_2$ est au choix un atome d'hydrogène; un radical benzyle; un radical phényle éventuellement substitué par au moins un alkyle en $C_1$-$C_{12}$; un radical alkyle en $C_1$-$C_{30}$ linéaire ou ramifié, comprenant éventuellement au moins un groupement cyclique, et éventuellement au moins un groupement aromatique, notamment en $C_1$-$C_{22}$, voire en $C_2$-$C_{16}$, comprenant éventuellement 1 à 4 hétéroatomes

choisis parmi O, N et S. On peut notamment citer les radicaux méthyle, éthyle, propyle, benzyle, éthylhexyle, lauryle, stéaryle, béhényle.

Parmi les monomères de formule (I) préférés, on peut citer :

- le (méth)acrylate de poly(éthylène glycol) dans lequel $R_1$ = H ou $CH_3$ ; Z = C(=O)-O- ; $R_2$ = H ; avec n = 2 à 200 ;
- le (méth)acrylate de méthyl-poly(éthylène glycol), aussi appelé (méth)acrylate de méthoxy-poly(éthylèneglycol), dans lequel $R_1$ = H ou $CH_3$ ; Z = C(=O)-O- ; et $R_2$=$CH_3$ ; avec n=2 à 200 ;
- les (méth)acrylate d'alkyl-poly(éthylène glycol) dans lequel $R_1$ = H ou $CH_3$ ; Z = C(=O)-O- ; et $R_2$ = alkyl en $C_1$-$C_{30}$ ; avec n = 2 à 200 ;
- les (méth)acrylates de phényl-poly(éthylène glycol), aussi appelé (méth)acrylate de poly(éthylène glycol) phényl éther, dans lequel $R_1$ = H ou $CH_3$ ; Z = C(=O)-O- ; et $R_2$ = phényle ; avec n = 2 à 200.

Les monomères de formule (I) tout particulièrement préférés peuvent être choisis dans le groupe comprenant les (méth)acrylates de poly(éthylène glycol) et les (méth)acrylates de méthyl-poly(éthylène glycol), de préférence ceux ayant une masse molaire compris entre 80 et 8000 g/mol, notamment entre 300 et 2000 g/mol.

Parmi les monomères commerciaux, on peut citer :

- les méthacrylates de polyéthylène glycol 8000 ou 4000 commercialisés par Monomer & Polymère Dajac laboratories ;
- les méthacrylates de poly(éthylène glycol), de masse molaire 5000 g/mol, disponibles chez EVONIK sous la dénomination commerciale VISIOMER® ;
- les méthacrylates de hydroxy-poly(éthylène glycol) commercialisés par CLARIANT sous la dénomination commerciale POLYGLYKOL® MA

Le ou les monomères cationiques présentant une fonction ammonium quaternaire pouvant être utilisés dans le cadre de l'invention peuvent être choisis, notamment parmi les monomères du type acrylamide, acrylique, vinylique, allylique ou maléique possédant une fonction ammonium quaternaire. On peut citer, en particulier et de façon non limitative, l'acrylate de diméthylaminoéthyle (ADAME), et le méthacrylate de diméthylaminoéthyle (MADAME) quaternisés, le chlorure de diméthyldiallylammonium (DADMAC), le chlorure d'acrylamido propyltriméthyl ammonium (APTAC) et le chlorure de methacrylamido propyltrimethyl ammonium (MAPTAC).

Selon un mode de réalisation particulier, le copolymère objet de la présente invention comprend également au moins un monomère non ionique distinct de celui correspondant à la formule (I) ci-dessus. De manière avantageuse, ce monomère non ionique additionnel représente moins de 25% en masse du copolymère, avantageusement de 5 à 25%.

Ainsi, selon un mode de réalisation particulier, le copolymère objet de l'invention peut être constitué de, en pourcentage en masse par rapport à la masse totale du copolymère :

- 5 à 45% d'au moins un monomère cationique dont la cationicité provient exclusivement de fonction(s) ammonium quaternaire ;
- 50 à 95% d'au moins un monomère de formule (I) ; avantageusement de 55 à 95% ;
- 5 à 25% d'au moins un monomère non ionique distinct du monomère de formule (I).

Le ou les monomères non ioniques pouvant être utilisés dans le cadre de l'invention peuvent être choisis, notamment, dans le groupe comprenant les monomères vinyliques solubles dans l'eau. Des monomères préférés appartenant à cette classe sont, par exemple, l'acrylamide, le méthacrylamide, le N-isopropylacrylamide, le N,N-diméthylacrylamide et le N-méthylolacrylamide. Egalement, peuvent être utilisés la N-vinylformamide, le N-vinyl acetamide, la N-vinylpyridine et la N-vinylpyrrolidone, l'acryloyl morpholine (ACMO) et la diacétone acrylamide. Un monomère non ionique préféré est l'acrylamide.

Selon certains modes de réalisation, en plus des monomères ci-dessus, le(s) copolymères hydrosolubles peuvent également comprendre un ou plusieurs monomères hydrophobes choisis, notamment, parmi les monomère de type acrylamide, acrylique, vinylique, allylique ou maléique possédant une fonction hydrophobe pendante choisis préférentiellement parmi les dérivés de l'acrylamide comme les N-alkylacrylamides, par exemple, le diacétone acrylamide, le N-tert-butylacrylamide, l'octylacrylamide, et les N,N-dialkylacrylamides comme le N,N-dihexylacrylamide et les dérivés d'acide acrylique comme les alkyl acrylates et méthacrylates. Egalement peuvent être utilisés les dérivés de monomères vinyliques comme la N-vinylformamide, le N-vinyl acétamide, la N-vinylpyridine, et le N-vinylimidazole.

De manière générale, les polymères de l'invention ne nécessitent pas de développement de procédé de polymérisation particulier. En effet, ils peuvent être obtenus selon toutes les techniques de polymérisation bien connues par l'homme de métier. Il peut notamment s'agir de polymérisation en solution ; polymérisation en gel ; polymérisation par précipitation ; polymérisation en émulsion (aqueuse ou inverse) ; polymérisation en suspension ; ou de polymérisation micellaire.

Le polymère peut se présenter sous forme liquide ou solide lorsque sa préparation inclut une étape de séchage tel que le spray drying, le séchage sur tambour ou encore le séchage micro ondes.

Comme déjà dit, par rapport aux polymères de l'art antérieur, le polymère mis au point par la Demanderesse présente une compatibilité améliorée avec des espèces anioniques.

Par le terme « espèces anioniques », on entend tous les éléments macromoléculaires, ayant un caractère anionique, couramment présents dans les formulations de type cosmétique, détergent ou autre.

Par conséquent, la présente invention concerne également l'utilisation du copolymère décrit ci-avant dans une formulation cosmétique ou détergente.

De manière non limitative, ces espèces ioniques peuvent être :

(i) Les tensioactifs anioniques parmi lesquels on peut citer, seuls ou mélangés, les sels (en particulier les sels de métaux alcalins, notamment les sels de sodium, les sels d'ammonium, les sels d'aminés, les sels d'aminoalcools ou les sels de magnésium) des composés suivants : les alkylsulfates, les alkyléthersulfates, les alkylamidoéthersulfates, les alkylarylpolyéthersulfates, les monoglycérides sulfates, les alkylsulfonates, les alkylphosphates, les alkylamidesulfonates, les alkylarylsulfonates, les alpha-oléfine-sulfonates, les paraffinesulfonates, les alkylsulfosuccinates, les alkyléthersulfosuccinates, les alkylamide- sulfosuccinates, les alkylsulfosuccinamates; les alkylsulfoacétates, les alkylétherphosphates, les acylsarcosinates, les acyliséthionates, et les N-acyltaurates. Le radical alkyle ou acyle de tous ces différents composés comporte de préférence de 8 à 24 atomes de carbone, alors que le radical aryle désigne de préférence un groupement phényle ou benzyle.
On peut également citer les sels d'acides gras tels que les sels des acides oléique, ricinoléique, palmitique, stéarique, les sels d'acides d'huile de coprah ou d'huile de coprah hydrogénée; les sels d'acyl-lactylates dont le radical acyle comporte 8 à 20 atomes de carbone; les sels d'acides d'alkyl D galactoside uroniques ainsi que les sels d'acides alkyl ($C_6$-$C_{24}$) éther carboxyliques polyoxyalkylénés, les sels d'acides alkyl($C_6$-$C_{24}$)aryl éther carboxyliques polyoxyalkylénés, les sels d'acides alkyl($C_6$-$C_{24}$) amido éther carboxyliques polyoxyalkylénés, en particulier ceux comportant de 2 à 50 groupements oxyde d'éthylène, et leurs mélanges.

(ii) Les polyelectrolytes anioniques comprenant au moins un monomère présentant une fonctionnalité acrylique, vinylique, maléique, fumarique, ou allylique et contenant un groupe carboxy, phosphonate, sulfonate, ou un autre groupe à charge anionique. Il peut notamment s'agir de : l'acide acrylique, l'acide méthacrylique, l'acide itaconique, l'acide crotonique, l'acide maléique, l'acide fumarique, et les monomères de type acide fort présentant par exemple une fonction de type acide sulfonique ou acide phosphonique tels que l'acide 2-acrylamido-2-méthylpropane sulfonique, l'acide vinylsulfonique, l'acide vinylphosphonique, l'acide allylsulfonique, l'acide allylphosphonique. Il peut également s'agir des polyélectrolytes à base de silicones comprenant un ou des groupes carboxylate, sulfate, sulfonate, phosphate ou phosphonate, ou leurs dérivés.

(iii) Les polymères naturels ayant un caractère anionique, pouvant être choisis dans le groupe comprenant les polysaccharides tels que la cellulose, l'amidon, la gomme de guar, la gomme de guar hémicellulose, la gomme arabique, le glucomannane, la gomme de caroube, le pullulane, le curdane, la gomme de xanthane, la gomme de gellane, la gomme de Carraghénane, la gomme de dextrane, la gomme adragante, la gomme de welane, la gomme rhamsane, l'acide hyaluronique, l'inuline, la pectine, la lignine, la chitine, l'alginate, l'agar-agar ou leurs dérivés.

L'invention et les avantages qui en découlent ressortiront mieux des figures et exemples suivants donnés afin d'illustrer l'invention et non de manière limitative.

## EXEMPLES DE REALISATION DE L'INVENTION

A/ Préparation des copolymères :

1/ Copolymère DADMAC / PEG 2000 MA (75/25) (Polymère A)

Le polymère A comprend 75 mol% de monomère DADMAC et 25 mol% de méthacrylate de polyéthylèneglycol, soit 20 % en masse de DADMAC et 80 % en masse de méthacrylate de polyéthylèneglycol.

Dans un réacteur équipé d'un système d'agitation mécanique, d'un condenseur, d'un thermomètre et d'une arrivée d'azote, on charge :

122 g de DADMAC (Flocryl, SNF)
585 g de PEG 2000 MA (Polyglykol® MA 2000, Clariant). Il s'agit d'un méthacrylate de polyéthylèneglycol dont la masse molaire est de 2000 g/mol.

1,75 g d'hypophosphite de sodium
261 g d'eau
0,03 g d'EDTA (éthylène diamine tétraacétique)

Le milieu réactionnel est désoxygéné avec un flux d'azote, et chauffé à 80°C.

Séparément, on prépare une solution d'initiateur en introduisant 0,35 g de 2,2' azobis(2-amidinopropane) dihydrochloride (V50, Wako) dans 30 g d'eau.

Quand la température du milieu a atteint 80°C, on démarre l'ajout progressif de la solution d'initiateur. La solution est ajoutée pendant 180 minutes, puis le milieu est maintenu à 80°C pendant 120 minutes supplémentaires pour compléter la polymérisation.

On laisse le mélange revenir à température ambiante, puis on ajuste le pH entre 5 et 7 en utilisant une solution aqueuse de NaOH ou d'acide citrique à 50% en masse.

Le produit obtenu est une solution liquide dont la concentration en polymère est de 40% en masse par rapport à la masse de la solution. La solution présente une viscosité de 300 cps (Brookfield LVT, module 3, 30 rpm). Le polymère A présente une structure peigne. Sa densité de charge cationique est de 1,15 meq/g.

2/ Copolymère DADMAC / PEG 2000 MA (98/2) (Polymère B) (ART ANTERIEUR)

Le polymère B comprend 98 mol% de monomère DADMAC et 2 mol% de méthacrylate de polyéthylèneglycol, soit 80 % en masse de DADMAC et 20% en masse de méthacrylate de polyéthylèneglycol.

En utilisant la même procédure que celle décrite dans l'exemple 1, on prépare un copolymère DADMAC / PEG 2000 MA contenant 2 mol% de PEG 2000 MA. Dans ce cas, les quantités suivantes sont utilisées :

499 g de DADMAC (Flocryl, SNF)
146 g PEG 2000 MA (Polyglykol® MA 2000, Clariant)
3,55 g d'hypophosphite de sodium
315 g d'eau
0,03 g d'EDTA
1,1g de azobis(2-amidinopropane) dihydrochloride (V50, Wako) dans 30 g d'eau

Le produit obtenu est un liquide présentant une concentration en polymère de 40% en masse par rapport à la masse du produit, et une viscosité de 1700 cps (Brookfield LVT, module 3, 30 rpm). Le polymère présente une structure peigne. Sa densité de charge cationique est de 4,26 meq/g.

3/ Homopolymère de DADMAC (Polymère C) (ART ANTERIEUR)

En utilisant la même procédure que celle décrite dans l'exemple 1, on prépare un homopolymère de DADMAC. Dans ce cas, les quantités suivantes sont utilisées :

625 g de DADMAC (Flocryl, SNF)
4,36 g d'hypophosphite de sodium
339 g d'eau
0,03g d'EDTA
1,34 g de V50 dans 30 g d'eau

Le produit obtenu est une solution liquide dont la concentration en polymère est de 40% en masse par rapport à la masse de la solution. La solution présente une viscosité de 200 cps (Brookfield LVT, module 3, 30 rpm). Le polymère C a une structure peigne. Sa densité de charge cationique est de 6,1 meq/g.

B/ Etude de la compatibilité entre les polymères et des polyéléctrolytes anioniques

Méthode :

La compatibilité entre des polymères cationiques et des polyélectrolytes anioniques est évaluée par titration.

La méthode suivante est utilisée :

- 50 g d'une solution aqueuse à 20% en masse en tensioactif anionique (TEA lauryl sulfate, TEA = triethanol amine) est préparée et mise sous agitation dans un bécher en verre.

- sous agitation, on réalise différents ajouts de polymère cationique et on observe l'apparence de la solution ainsi préparée.

On détermine la masse maximale de produit cationique que l'on peut ajouter avant apparition d'un précipité ou d'un trouble traduisant la précipitation.

La quantité de polymère cationique introduite est alors reliée à une charge cationique apportée dans le système.

Résultats :

La compatibilité entre les polymères (A, B, C et polyquaternium 7) et le TEA lauryl sulfate (solution à 20% en masse) a été évaluée. Les résultats de la titration sont présentés dans le tableau ci-après :

| Polymère cationique | Densité de charge du polymère (meq/g) | Tensioactif anionique (solution à 20%) | Quantité de polymère ajoutée (g de polymère sec) | Apparence | Cationicité totale (meq) |
|---|---|---|---|---|---|
| Polymère A | 1,15 | TEA lauryl sulfate | 0,45 | Solution translucide | 0,5 |
| Polymère A | 1,15 | TEA lauryl sulfate | 1,00 | Solution translucide | 1,2 |
| Polymère A | 1,15 | TEA lauryl sulfate | 2,40 | Solution translucide | 2,8 |
| Polymère A | 1,15 | TEA lauryl sulfate | 4,80 | Solution translucide | 5,5 |
| Polymère B | 4,26 | TEA lauryl sulfate | 0,20 | Solution translucide | 0,9 |
| Polymère B | 4,26 | TEA lauryl sulfate | 0,50 | Solution translucide | 2,1 |
| Polymère B | 4,26 | TEA lauryl sulfate | 1,00 | Léger voile blanchâtre | 4,3 |
| Polymère B | 4,26 | TEA lauryl sulfate | 1,30 | Léger voile blanchâtre | 5,5 |
| Polymère C | 6,2 | TEA lauryl sulfate | 0,20 | Particules insolubles | 1,2 |
| Polymère C | 6,2 | TEA lauryl sulfate | 0,45 | Particules insolubles | 2,8 |
| Polymère C | 6,2 | TEA lauryl sulfate | 0,90 | Particules insolubles | 5,6 |
| Polyquaternium 7* | 1,4** | TEA lauryl sulfate | 1,00 | Solution translucide | 1,4 |
| Polyquaternium 7* | 1,4** | TEA lauryl sulfate | 1,50 | Solution translucide | 2,1 |
| Polyquaternium 7* | 1,4** | TEA lauryl sulfate | 2,00 | Particules insolubles | 2,8 |
| * polyquaternium 7 (Merquat 550, Nalco) : Copolymère DADMAC/AM (30 mol% / 70 mol %), AM = acrylamide<br>** densité de charge mesurée | | | | | |

A cationicité totale équivalente (autour de 5,5, valeur atteinte après addition d'une quantité de polymère variant selon leur nature), les solutions contenant le polymère A selon l'invention, présentent une apparence translucide alors que les solutions comprenant les polymères B et C contiennent des particules de polymères insolubles. Le polyquaternium

7 a une densité de charge comprise dans la fourchette revendiquée. Toutefois après avoir ajouté 2 g de polymère dans la solution il y a apparition de particules insolubles.

## Revendications

**1.** Copolymère hydrosoluble comprenant, en masse par rapport à la masse totale du copolymère :

- 5 à 45% d'au moins un monomère cationique vinylique possédant une fonction ammonium quaternaire ;
- 50 à 95% d'au moins un monomère de formule (I) ;

(I) 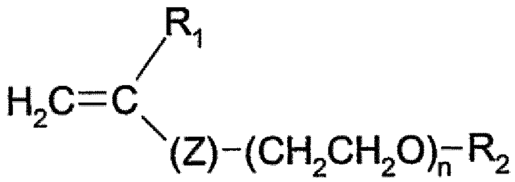

dans laquelle :

- $R_1$ est un atome d'hydrogène ou un radical méthyle ;
- Z est un groupement divalent -C(=O)-O-, ou -C(=O)-NH- ;
- n est un nombre entier compris entre 2 et 200 ;
- $R_2$ est un atome d'hydrogène ou un radical carboné, saturé ou insaturé, éventuellement aromatique, linéaire, ramifié ou cyclique, comprenant de 1 à 30 atomes de carbone, et de 0 à 4 hétéroatomes choisis dans le groupe comprenant O, N, et S ;

le copolymère présentant une densité de charge cationique comprise entre 0,3 et 2,6 meq/g.

**2.** Copolymère selon la revendication 1, ***caractérisé*** **en ce que** le copolymère présente une densité de charge cationique comprise entre 0,5 et 1,5 meq/g.

**3.** Copolymère selon l'une des revendications précédentes, ***caractérisé*** **en ce que** le monomère de formule (I) est choisi dans le groupe comprenant :

- le (méth)acrylate de poly(éthylène glycol) dans lequel $R_1$ = H ou $CH_3$ ; Z = C(=O)-O ; $R_2$ = H ; n = 2 à 200 ;
- le (méth)acrylate de méthyl-poly(éthylène glycol), aussi appelé (méth)acrylate de méthoxy-poly(éthylèneglycol), dans lequel $R_1$ = H ou $CH_3$ ; Z = C(=O)-O ; $R_2$ = $CH_3$ ; n = 2 à 200 ;
- les (méth)acrylate d'alkyl-poly(éthylène glycol) dans lequel $R_1$ = H ou $CH_3$ ; Z = C(=O)-O ; $R_2$ = alkyl en $C_1$-$C_{30}$ ; n = 2 à 200 ;
- les (méth)acrylates de phényl-poly(éthylène glycol), aussi appelé (méth)acrylate de poly(éthylène glycol) phényl éther, dans lequel $R_1$ = H ou $CH_3$ ; Z = C(=O)-O ; $R_2$ = phényle ; n = 2 à 200.

**4.** Copolymère selon la revendication 3, ***caractérisé*** **en ce que** le monomère de formule (I) est choisi dans le groupe comprenant les (méth)acrylates de poly(éthylène glycol) et les (méth)acrylates de méthyl-poly(éthylène glycol), présentant une masse molaire comprise entre 80 et 8000 g/mol.

**5.** Copolymère selon l'une des revendications précédentes, ***caractérisé*** **en ce que** le monomère cationique est choisi dans le groupe comprenant l'acrylate de diméthylaminoéthyl (ADAME) quaternisé, le méthacrylate de diméthylaminoéthyle (MADAME) quaternisé, le chlorure de diméthyldiallylammonium (DADMAC), le chlorure d'acrylamido propyltriméthyl ammonium (APTAC) et le chlorure de methacrylamido propyltrimethyl ammonium (MAPTAC).

**6.** Copolymère selon l'une des revendications précédentes, ***caractérisé*** **en ce que** le copolymère comprend en outre au moins un monomère non ionique distinct du monomère de formule (I).

**7.** Copolymère selon la revendication 6, ***caractérisé*** **en ce que** le monomère non ionique représente moins de 25% en masse du copolymère.

**8.** Copolymère selon la revendication 6, ***caractérisé* en ce que** le monomère non ionique est choisi dans le groupe comprenant l'acrylamide et le méthacrylamide, le N-isopropylacrylamide, le N,N-diméthylacrylamide, le N-méthylolacrylamide, la N-vinylformamide, le N-vinyl acetamide, la N-vinylpyridine, la N-vinylpyrrolidone, l'acryloyl morpholine (ACMO), et la diacétone acrylamide.

**9.** Copolymère selon l'une des revendications précédentes, ***caractérisé* en ce que** la totalité des charges cationiques du copolymère provient de fonctions ammonium quaternaire.

**10.** Copolymère selon l'une des revendications précédentes, ***caractérisé* en ce qu'**il est constitué de, en pourcentage en masse par rapport à la masse totale du copolymère :

- 5 à 45% d'au moins un monomère cationique dont la cationicité provient exclusivement de fonction(s) ammonium quaternaire ;
- 50 à 95% d'au moins un monomère de formule (I) ;
- 5 à 25% d'au moins un monomère non ionique distinct du monomère de formule (I).

**11.** Copolymère selon l'une des revendications 1 à 5, ***caractérisé* en ce qu'**il est constitué de, en pourcentage en masse par rapport à la masse totale du copolymère :

- 5 à 45% d'au moins un monomère cationique dont la cationicité provient exclusivement de fonction(s) ammonium quaternaire ;
- 55 à 95% d'au moins un monomère de formule (I).

**12.** Utilisation du copolymère objet de l'une des revendications précédentes, dans une formulation cosmétique ou détergente.

**Patentansprüche**

**1.** Wasserlösliches Copolymer, das, in Gewichtsprozent bezogen auf das Gesamtgewicht des Copolymers umfasst:

- mindestens 5 bis 45 % eines kationischen Vinyl-Monomers, das eine quaternäre Ammoniumfunktion besitzt;
- mindestens 50 bis 95 % eines Monomers der Formel (I);

(I) 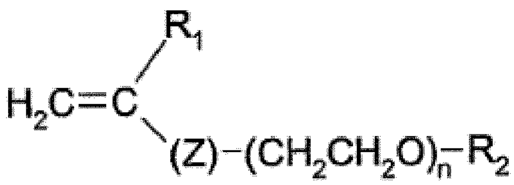

in der:

- $R_1$ ein Wasserstoffatom oder ein Methylradikal ist;
- Z eine divalente Gruppe-C (= O)-O-oder-C (= O)-NH ist-;
- n ist eine ganze Zahl zwischen 2 und 200;
- $R_2$ ist ein Wasserstoffatom oder ein Kohlenstoffradikal, gesättigt oder ungesättigt, gegebenenfalls aromatisch, linear, verzweigt oder zyklisch, zwischen 1 und 30 Kohlenstoffatomen und zwischen 0 und 4 Heteroatome umfassend ausgewählt aus der Gruppe bestehend aus O, N und S;
- das Copolymer weist eine kationische Ladungsdichte von 0,3 bis 2,6 meq/g auf.

**2.** Copolymer gemäß Anspruch 1, ***dadurch gekennzeichnet, dass*** das Copolymer eine kationische Ladungsdichte zwischen 0,5 und 1,5 meq/g aufweist.

**3.** Copolymer nach einem der vorhergehenden Ansprüche, ***dadurch gekennzeichnet, dass*** das Monomer der Formel (1) aus der Gruppe ausgewählt wird, die umfasst:

- Poly(ethylenglycol)-(Meth)acrylat in dem $R_1$= H oder $CH_3$; Z = C(=O)-O; $R_2$ = H ; n = 2 bis 200;

- Methyl-Poly(ethylenglycol)-(Meth)acrylat auch genannt Methoxy-(ethylenglycol)-(Meth)acrylat, in dem $R_I$ = H oder $CH_3$; Z = C(=O)-O; $R_2$ = $CH_3$; n = 2 bis 200 ;
- (Alkyl-Poly(ethylenglycol)-(Meth)acrylat in denen $R_1$ = H oder $CH_3$; Z = C(=O)-O; $R_2$ = Alkyl in $C_1$-$C_{30}$; n = 2 bis 200;
- Phenyl-Poly(ethylenglycol)-(Meth)acrylat, auch genannt Phenylether-Poly(ethylenglycol)-(Meth)acrylat, in dem $R_1$ = H oder $CH_3$; Z = C(=O)-O; $R_2$ = Phenyl; n = 2 bis 200.

**4.** Copolymer gemäß Anspruch 3, ***dadurch gekennzeichnet, dass*** das Monomer der Formel (1) aus der Gruppe ausgewählt wird, die die Poly(ethylenglycol)-(Meth)acrylate und die Methyl-Poly(ethylenglycol)-(Meth)acrylate umfasst, die eine molare Masse zwischen 80 und 8000 g/mol aufweisen.

**5.** Copolymer nach einem der vorhergehenden Ansprüche, ***dadurch gekennzeichnet, dass*** das kationische Monomer aus der Gruppe ausgewählt wird, die umfasst quaternisiertes Dimethylaminoethylacrylat (DMAEA), quaternisiertes Dimethylaminoethyl-Methacrylat (DMAEMA), Diallyldimethylammoniumchlorid (DADMAC), Acrylamidopropyltrimethylammoniumchlorid (APTAC) und Methacrylamidopropyltrimethylammoniumchlorid (MAPTAC).

**6.** Copolymer nach einem der vorhergehenden Ansprüche, ***dadurch gekennzeichnet, dass*** das Copolymer außerdem mindestens ein nicht-ionisches Monomer enthält, das von dem Monomer der Formel (1) verschieden ist.

**7.** Copolymer nach einem der vorhergehenden Ansprüche, ***dadurch gekennzeichnet, dass*** das nicht-ionische Monomer mindestens 25 Massen% des Copolymers darstellt.

**8.** Copolymer nach einem der vorhergehenden Ansprüche, ***dadurch gekennzeichnet, dass*** das nicht-ionische Monomer aus der Gruppe ausgewählt wird, die umfasst Acrylamid und Methacrylamid, N-Isoporpylacrylamid, N,N-Dimetlrylacrylamid, N-Methylolacrylamid, N-Vinylformamid, N-Vinyl-Acetamid, N-Vinylpyridin, N-Vinylpyrrolidon, Acryloyl Morpholin (ACMO) und Diaceton-Acrylamid.

**9.** Copolymer nach einem der vorhergehenden Ansprüche, **dadurch gekennzeichnet, dass** alle kationischen Ladungen des Copolymers aus quaternären Ammoniumfunktionen stammen.

**10.** Copolymer nach einem der vorhergehenden Ansprüche, ***dadurch gekennzeichnet, dass*** es in Masseprozent bezogen auf die Gesamtmasse des Copolymers besteht aus:

- mindestens 5-45 % eines kationischen Monomers, dessen Kationizität ausschließlich aus quaternären Ammoniumfunktionen stammt;
- mindestens 50 bis 95 % eines Monomers der Formel (1);
- mindestens 5 bis 25 % eines nicht-ionischen Monomers, das von dem Monomer der Formel (1) verschieden ist.

**11.** Copolymer nach einem der Ansprüche 1 bis 5, ***dadurch gekennzeichnet, dass*** es in Masseprozent bezogen auf die Gesamtmasse des Copolymers besteht aus:

- mindestens 5-45 % eines kationischen Monomers, dessen Kationizität ausschließlich aus quaternären Ammoniumfunktionen stammt;
- 55 bis 95 % mindestens eines Monomers der Formel (1);

**12.** Verwendung des Copolymers, das Gegenstand eines der vorhergehenden Ansprüche ist, in einer Kosmetika-oder Waschmittel-Formulierung.

## Claims

**1.** A water-soluble copolymer comprising, by mass relative to the total mass of the copolymer:

- 5 to 45% of at least one vinyl cationic monomer having a quaternary ammonium function;
- 50 to 95% of at least one monomer of formula (I);

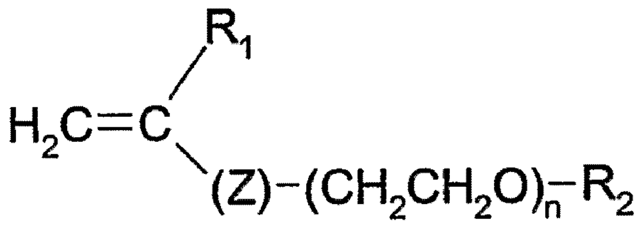

(I)

in which:

- $R_1$ is a hydrogen atom or a methyl radical;
- Z is a divalent group -C(=O)-O-, or -C(=O)-NH-;
- n is an integer between 2 and 200;
- $R_2$ is a hydrogen atom or a carbon-containing radical which is saturated or unsaturated, optionally aromatic, linear, branched or cyclic, comprising 1 to 30 carbon atoms, and from 0 to 4 heteroatoms chosen from the group comprising O, N and S;

the copolymer having a cationic charge density of between 0.3 and 2.6 meq/g.

2. The copolymer as claimed in claim 1, ***characterised* in that** the copolymer has a cationic charge density of between 0.5 and 1.5 meq/g.

3. The copolymer as claimed in either of the preceding claims, ***characterised* in that** the monomer of formula (I) is chosen from the group comprising:

- poly(ethylene glycol) (meth)acrylate in which $R_1$ = H or $CH_3$; Z = C(=O)-O-; $R_2$ = H; n = 2 to 200;
- methyl-poly(ethylene glycol) (meth)acrylate, also called methoxy-poly(ethylene glycol) (meth)acrylate, in which $R_1$ = H or $CH_3$; Z = C(=O)-O-; $R_2$ = $CH_3$; n = 2 to 200;
- alkyl-poly(ethylene glycol) (meth)acrylates in which $R_1$ = H or $CH_3$; Z = C(=O)-O-; $R_2$ = $C_1$-$C_{30}$ alkyl; n = 2 to 200;
- phenyl-poly(ethylene glycol) (meth)acrylates, also called poly(ethylene glycol) phenyl ether (meth)acrylate, in which $R_1$ = H or $CH_3$; Z = C(=O)-O-; $R_2$ = phenyl; n = 2 to 200.

4. The copolymer as claimed in claim 3, ***characterised* in that** the monomer of formula (I) is chosen from the group comprising poly(ethylene glycol) (meth)acrylates and methyl-poly(ethylene glycol) (meth)acrylates, having a molar mass of between 80 and 8000 g/mol.

5. The copolymer as claimed in one of the preceding claims, ***characterised* in that** the cationic monomer is chosen from the group comprising the quaternized dimethylaminoethyl acrylate (ADAME), the quaternized dimethylaminoethyl methacrylate (MADAME), the dimethyldiallylammonium chloride (DADMAC), the acrylamidopropyl trimethyl ammonium chloride (APTAC) and the methacrylamido propyltrimethyl ammonium chloride (MAPTAC).

6. The copolymer as claimed in one of the preceding claims, ***characterised* in that** the copolymer also comprises at least one nonionic monomer distinct from the monomer of formula (I).

7. The copolymer as claimed in claim 6, ***characterised* in that** the nonionic monomer represents less than 25% by mass of the copolymer.

8. The copolymer as claimed in claim 6, ***characterised* in that** the nonionic monomer is chosen from the group comprising acrylamide, methacrylamide, N-isopropylacrylamide, N,N-dimethylacrylamide, N-methylolacrylamide, N-vinylformamide, N-vinylacetamide, N-vinylpyridine, N-vinylpyrrolidone, acryloylmorpholine (ACMO) and diacetone acrylamide.

9. The copolymer as claimed in one of the preceding claims, ***characterised* in that** all of the cationic charges of the copolymer come from quaternary ammonium functional groups.

**10.** The copolymer as claimed in one of the preceding claims, ***characterised* in that** it consists of, as percentage by mass relative to the total mass of the copolymer:

- 5 to 45% of at least one cationic monomer whose cationicity comes exclusively from one or more quaternary ammonium functional groups;
- 50 to 95% of at least one monomer of formula (I);
- 5 to 25% of at least one nonionic monomer distinct from the monomer of formula (I).

**11.** The copolymer as claimed in one of claims 1 to 5, ***characterised* in that** it consists of, as percentage by mass relative to the total mass of the copolymer:

- 5 to 45% of at least one cationic monomer whose cationicity comes exclusively from one or more quaternary ammonium functional groups;
- 55 to 95% of at least one monomer of formula (I).

**12.** The use of the copolymer which is the subject of one of the preceding claims, in a cosmetic or detergent formulation.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 372546 A
- JP 2002322219 A
- JP 2003055164 A
- JP 2000302649 A
- JP 7285831 A
- EP 1769011 A
- EP 1765893 A
- WO 2006013268 A
- AU 2004200189
- FR 2962034